(19) **Europäisches Patentamt**
**European Patent Office**
**Office européen des brevets**

(11) **EP 1 532 224 B1**

(12) **EUROPEAN PATENT SPECIFICATION**

(45) Date of publication and mention
of the grant of the patent:
**03.01.2007 Bulletin 2007/01**

(51) Int Cl.:
**C09K 11/78** (2006.01)

(21) Application number: **03792581.5**

(22) Date of filing: **14.08.2003**

(86) International application number:
**PCT/IB2003/003639**

(87) International publication number:
**WO 2004/018589 (04.03.2004 Gazette 2004/10)**

(54) **DEVICE FOR GENERATING RADIATION**

VORRICHTUNG ZUR ERZEUGUNG VON STRAHLUNG

DISPOSITIF POUR GENERER DES RAYONNEMENTS

(84) Designated Contracting States:
**AT BE BG CH CY CZ DE DK EE ES FI FR GB GR HU IE IT LI LU MC NL PT RO SE SI SK TR**

(30) Priority: **22.08.2002 DE 10238399**

(43) Date of publication of application:
**25.05.2005 Bulletin 2005/21**

(73) Proprietors:
- **Philips Intellectual Property & Standards GmbH**
**20099 Hamburg (DE)**
Designated Contracting States:
**DE**
- **Koninklijke Philips Electronics N.V.**
**5621 BA Eindhoven (NL)**
Designated Contracting States:
**AT BE BG CH CY CZ DK EE ES FI FR GB GR HU IE IT LI LU MC NL PT RO SE SI SK TR**

(72) Inventors:
- **JÜSTEL, Thomas,**
**Philips Intel.Prop. & Std. GmbH**
**52066 Aachen (DE)**

- **MAYR, Walter,**
**Philips Intel.Prop. & Std. GmbH**
**52066 Aachen (DE)**
- **SCHMIDT, Peter J.,**
**Philips Intel.Prop. & Std. GmbH**
**52066 Aachen (DE)**
- **BLANKEFORT, Helmut,**
**Philips Intel.Prop. & Std.GmbH**
**52066 Aachen (DE)**

(74) Representative: **Volmer, Georg**
**Philips Intellectual Property & Standards GmbH,**
**Postfach 50 04 42**
**52088 Aachen (DE)**

(56) References cited:
**EP-A- 0 383 388** **US-A- 4 088 599**

- **DATABASE WPI Section Ch, Week 200166 Derwent Publications Ltd., London, GB; Class L03, AN 2001-587434 XP002260974 & KR 2001 026 297 A (null) 6 April 2001 (2001-04-06)**

**Description**

The invention relates to a device for generating radiation by means of excimer discharge, equipped with an at least partly UV-transparent discharge vessel, the discharge space of which is filled with a gas filling, with means for igniting and maintaining an excimer discharge in the discharge spacer and with a coating comprising a light-emitting compound. The invention further relates to the use of the device and to a light-emitting compound.

Phosphors are materials capable of luminescence, which emit light when excited. Exciting energy forms that are possibilities here are electromagnetic radiation such as y rays, X-rays, UV light or visible light, corpuscular rays such as $\alpha$ rays or $\beta$ rays, electrical fields (electroluminescence) or mechanical effects (triboluminescence).

The use of phosphors is extremely varied and depends on the type of excitation and the wavelength of the emitted light. Thus, phosphors that luminesce on excitation with electron beams are used in, for example, television and computer screens, oscillograph tubes or image converters.

Phosphors that luminesce on excitation with X-rays are used to manufacture radioscopy screens and X-ray image amplifiers for medical purposes. The phosphors used must have base materials that are capable of strongly absorbing X-rays.

Phosphors that luminesce on excitation with UV radiation are used in, for example, gas discharge lamps or plasma screens.

The EP 0 383 388 discloses for example a phosphor composition of the formula $M_{2-3/2x-\frac{1}{2}y} Pr_x M'_y SiO_4$ wherein M is at least one of the elements Ca and Sr replaced with Ba and/or Mg, M' is at least one of the elements Li, Na, K and $0.004 \leq x \leq 0.10$ and $0 \leq y \leq 0.10$, used in a low pressure mercury vapour lamp.

Although suitable phosphors are available for virtually all application areas, there are still some in which the phosphors used are not yet optimal.

Furthermore, it is necessary to make phosphors available for new or special applications and for new technologies. One example of a special application is the use of xenon gas discharge lamps for disinfection purposes. Using a xenon gas discharge lamp emitting UV-C light, pathogenic microorganisms present in water, such as bacteria, fungi, viruses or protozoans, can be rendered harmless. A xenon gas discharge lamp for water disinfection is known from EP 1 048 620.

In accordance with DIN 5031-10, the maximum photobiologically effective UV radiation lies at around 265 nm.

It is therefore an object of the present invention to provide a device for generating radiation with an emission spectrum that is simple and can be optimally adjusted to suit the desired application.

It is, in particular, an object of the present invention to provide a device for generating radiation with an emission spectrum having a large overlap with DIN 5031-10.

This object is achieved by means of a device for generating radiation by means of excimer discharge, equipped with an at least partly UV-transparent discharge vessel, the discharge chamber of which is filled with a gas filling, with means for igniting and maintaining an excimer discharge in the discharge chamber and with a coating comprising a light-emitting compound of the following composition:

$$(Ca_{1-x-2y}Sr_x)Li_2Si_{1-z}Ge_zO_4:Ln_yM_y,$$

wherein Ln is a cation selected from the group $Ce^{3+}$, $Pr^{3+}$, $Sm^{3+}$, $Eu^{3+}$, $Gd^{3+}$, $Tb^{3+}$, $Dy^{3+}$, $Er^{3+}$, $Tm^{3+}$ and $Yb^{3+}$, and M is a cation selected from the group $Na^+$, $K^+$ and $Rb^+$, $0 \leq x \leq 0.1$, $0.001 \leq y \leq 0.2$ and $0 \leq z \leq 1$.

The light-emitting compounds activated with $Ce^{3+}$, $Pr^{3+}$, $Sm^{3+}$, $Eu^{3+}$, $Gd^{3+}$, $Tb^{3+}$, $Dy^{3+}$, $Er^{3+}$, $Tm^{3+}$ or $Yb^{3+}$ can all be efficiently excited using (V)UV light and have high absorption efficiencies, particularly at wavelengths in the range of xenon excimer radiation.

The device for generating radiation as claimed in claim 2 has an emission spectrum with emission maxima located at 255 and 265 nm, which is in the photobiologically effective range.

The invention also relates to the use of a device in accordance with the invention for disinfection purposes, in particular for the disinfection of water, air or surfaces.

The invention further relates to a light-emitting compound of the following composition:

$$(Ca_{1-x-2y}Sr_x)Li_2Si_{1-z}Ge_zO_4:Ln_yM_y,$$

wherein Ln is a cation selected from the group $Ce^{3+}$, $Pr^{3+}$, $Sm^{3+}$, $Eu^{3+}$, $Gd^{3+}$, $Tb^{3+}$, $Dy^{3+}$, $Er^{3+}$, $Tm^{3+}$ and $Yb^{3+}$, and M is a cation selected from the group $Na^+$, $K^+$ and $Rb^+$, $0 \leq x \leq 0.1$, $0.001 \leq y \leq 0.2$ and $0 \leq z \leq 1$.

The invention will be further described with reference to examples of embodiments shown in the drawings, to which, however, the invention is not restricted.

Fig. 1 shows, in longitudinal cross-section, a device for generating radiation.
Fig. 2 shows, in cross-section, a further device for generating radiation.
Fig. 3 shows, in cross-section, a yet further device for generating radiation.
Fig. 4 shows the excitation and emission spectrum of $Ca_{1-2y}Li_2SiO_4:Pr_yNa_y$ with 0.001 1 $\leq$ y $\leq$ 0.2.
Fig. 5 shows DIN 5031-10 and the emission spectrum of $Ca_{1-2y}Li_2SiO_4:Pr_yNa_y$ with 0.00 1 $\leq$ y $\leq$ 0.2.
Fig. 6 shows the excitation and emission spectrum of $Ca_{1-2y}Li_2SiO_4:Ce_yNa_y$ with 0.001 $\leq$ y $\leq$ 0.2.
Fig. 7 shows the excitation and emission spectrum of $Ca_{1-2y}Li_2SiO_4:Eu_yNa_y$ with 0.001 1 $\leq$ y $\leq$ 0.2.

A device in accordance with the invention for generating radiation by means of excimer discharge is equipped with an at least partly UV-transparent discharge vessel 1, the discharge space 2 of which is filled with a gas filling, and with a coating 3 comprising a light-emitting compound. In addition, the device is equipped with means for igniting and maintaining the excimer discharge.

A multiplicity of designs, such as panels, simple tubes, coaxial tubes, discharge tubes that are straight, U-shaped, circular, spiraled or cylindrical or of some other shape, may be used for the discharge vessel 1. The design hereby depends largely on the planned application of the device.

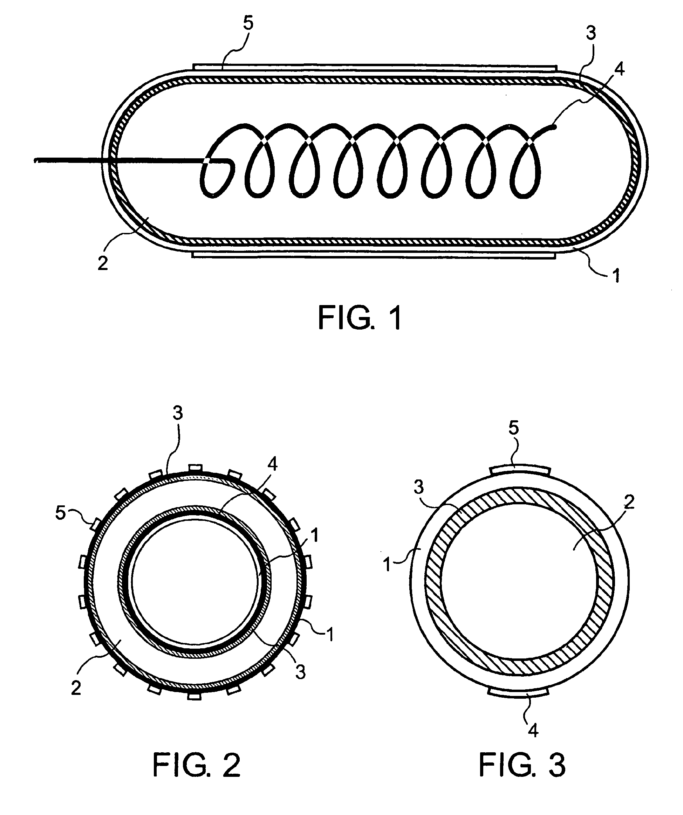

A typical design for a device for generating radiation is the shape as shown in Fig. 1. As the means for igniting and maintaining the excimer discharge, this design comprises *inter alia* electrodes of first and second kinds 4, 5. Inserted concentrically into the discharge vessel 1 is a spiraled wire. This forms the first, inner electrode 4 of the device. The outer glass is covered with narrow-gauge wire netting, forming the second, outer electrode 5. Discharge vessel 1 is sealed to be gas-tight. The discharge space 2 contains a gas filling and on the inner walls of discharge vessel 1 is a coating 3, which comprises a light-emitting compound. The two electrodes 4, 5 are connected to the two poles of an alternating current source. The electrode geometry together with the pressure in discharge vessel 1 and the gas composition are matched to the data for the alternating current source.

A different design, which is particularly suitable for disinfection purposes, is the coaxial shape in Fig. 2, carrying a medium to be disinfected, such as water or air, centrally. The discharge vessel 1 comprises two coaxial glass components with a gas-tight connection to a hollow sleeve. The annular clearance between the two coaxial glass components forms the discharge chamber 2 and is provided with a gas filling. The fluid medium for disinfection can flow through the inner tube, on the inner wall of which a transparent electrode of the first kind 4 is arranged. The medium to be disinfected may also be located outside the outer tube. The outer glass is covered with narrow-gauge wire netting, forming the outer, second electrode 5. The power supply is from an alternating current source connected to these two electrodes. The inner surfaces of the hollow sleeve have a coating 3 with a light-emitting compound.

In a further possible design as shown in Fig. 3, the device comprises a cylindrical discharge vessel 1 made of glass, with a gas filling, on the walls of which the electrode of the first kind 4 and the electrode of the second kind 5 are arranged externally, electrically insulated from each other. The strip-shaped electrodes 4, 5 extend over the entire length of discharge vessel 1, wherein their long sides face each other, leaving two gaps free. The coating 3 with the light-emitting substance is applied to the inner walls of discharge vessel 1.

The means for igniting and maintaining an excimer discharge comprise electrodes of first and second kinds 4, 5. Electrodes 4, 5 comprise a metal, for example aluminum or silver, a metal alloy or a transparent, conductive, inorganic compound, e.g. ITO. These may take the form of a coating, a bonded-on film, bonded-on strips of film, wire or wire netting.

Discharge vessel 1 is equipped with a gas filling of xenon, krypton, argon, neon, helium or a gas mixture with at least one of these gases. The gas filling will preferably contain oxygen-free xenon or a gas mixture containing xenon.

Coating 3 comprises a light-emitting compound with the following composition:

$$(Ca_{1-x-2y}Sr_x)Li_2Si_{1-z}Ge_zO_4:Ln_yM_y,$$

wherein Ln is a cation selected from the group $Ce^{3+}$, $Pr^{3+}$, $Sm^{3+}$, $Eu^{3+}$, $Gd^{3+}$, $Tb^{3+}$, $Dy^{3+}$, $Er^{3+}$, $Tm^{3+}$ and $Yb^{3+}$,
and M is a cation selected from the group $Na^+$, $K^+$ and $Rb^+$,
$0 \leq x \leq 0.1$, $0.001 \leq y \leq 0.2$ and $0 \leq z \leq 1$.

The host lattice $(Ca_{1-x-2y}Sr_x)Li_2Si_{1-z}Ge_zO_4$ of the light-emitting compound crystallizes in a tetragonal crystal system. Through doping with trivalent metal cations, phosphors having different emission properties depending on the metal cation used are obtained. The trivalent metal cations are incorporated into the divalent calcium positions. For charging compensation, monovalent cations are also incorporated into the crystal lattice. Also advantageous in the case of this host lattice is the fact that the dopants can be incorporated into the host lattice at high concentrations.

The light-emitting compound preferably has a composition of:

$Ca_{1-2y}Li_2SiO_4:Pr_yNa_y$, $Ca_{1-2y}Li_2SiO_4:Ce_yNa_y$, $Ca_{1-2y}Li_2SiO_4:Eu_yNa_y$, $Ca_{1-2y}Li_2SiO_4:Tb_yNa_y$ or $Ca_{1-2y}Li_2SiO_4:Nd_y$-$Na_y$ with $0.001 \leq y \leq 0.2$ in each case. The light-emitting substance is selected depending on the application. If the device is to be used for disinfection purposes, e.g. for disinfection of water, air or surfaces, $Ca_{1-2y}Li_2SiO_4:Pr_yNa_y$

with $0.001 \leq y \leq 0.2$ is preferably used as the light-emitting substance.

Alternatively, coating 3 may also comprise an organic or inorganic binder or binder composition. Furthermore, coating 3 or the light-emitting compound itself may also be protected against the discharge attack with a protective layer.

If an alternating voltage is applied to the electrodes 4, 5, a silent electrical charge is ignited in the preferred, xenon-containing filler gas. As a result, xenon excimers, i.e. molecules that are stable only in the excited state, are formed in the plasma.

$$Xe + Xe^{\bullet} = Xe_2^{\bullet}$$

The excitation energy is released again as UV radiation with a wavelength of $\lambda = 140$ to 190 nm. This conversion of electron energy into UV radiation takes place extremely efficiently. The UV photons generated are absorbed by the light-emitting compound, and some of the excitation energy is released again in an area of the spectrum with a longer wavelength. The absorption coefficient of the light-emitting compounds activated with $Ce^{3+}$, $Pr^{3+}$, $Sm^{3+}$, $Eu^{3+}$, $Gd^{3+}$, $Tb^{3+}$, $Dy^{3+}$, $Er^{3+}$, $Tm^{3+}$ or $Yb^{3+}$ is especially great for the wavelengths in the area of xenon radiation, and the quantum yield is high. The host lattice influences the precise position of the energy level of the activator cation and, as a result, influences the emission spectrum.

In order to produce the light-emitting compound, appropriate quantities of the starting compounds for the host lattice, $Li_2SiO_3$, $Li_2GeO_3$, $CaCO_3$ and $SrCO_3$, are mixed in water with the appropriate quantities of a salt of the trivalent metal cation $Ln^{3+}$ and a salt of the monovalent charge compensator $M^+$. The water is subsequently removed. The reaction mixture obtained is dried and annealed multiple times at temperatures between 700 and 900°C.

Preferably used as salts of the trivalent metal cations are the nitrates $Ln(NO_3)_3$ with Ln selected from the group $Ce^{3+}$, $Pr^{3+}$, $Sm^{3+}$, $Eu^{3+}$, $Gd^{3+}$, $Tb^{3+}$, $Dy^{3+}$, $Er^{3+}$, $Tm^{3+}$ and $Yb^{3+}$. Preferably used as salts of the charge compensator are the carbonates $M_2CO_3$ with M selected from the group $Na^+$, $K^+$ and $Rb^+$.

The light-emitting compound is applied to the vessel walls of the discharge vessel 1 using a flow coating method. The coating suspensions for the flow coating method contain water or an organic compound such as butyl acetate as the solvent. The suspension is stabilized and influenced in its rheological properties by the addition of auxiliary agents such as stabilizers, liquefiers or cellulose derivatives. The suspension of the light-emitting compound is applied as a thin layer to the vessel walls of discharge vessel 1, is dried and baked at 600°C. Discharge vessel 1 is then evacuated in order to remove all gaseous impurities, especially oxygen. The vessel is then filled with xenon with a gas pressure of approximately 200 to 300 mbar, and sealed.

The uses of the device described are varied, and depend primarily on the emission spectrum of the device.

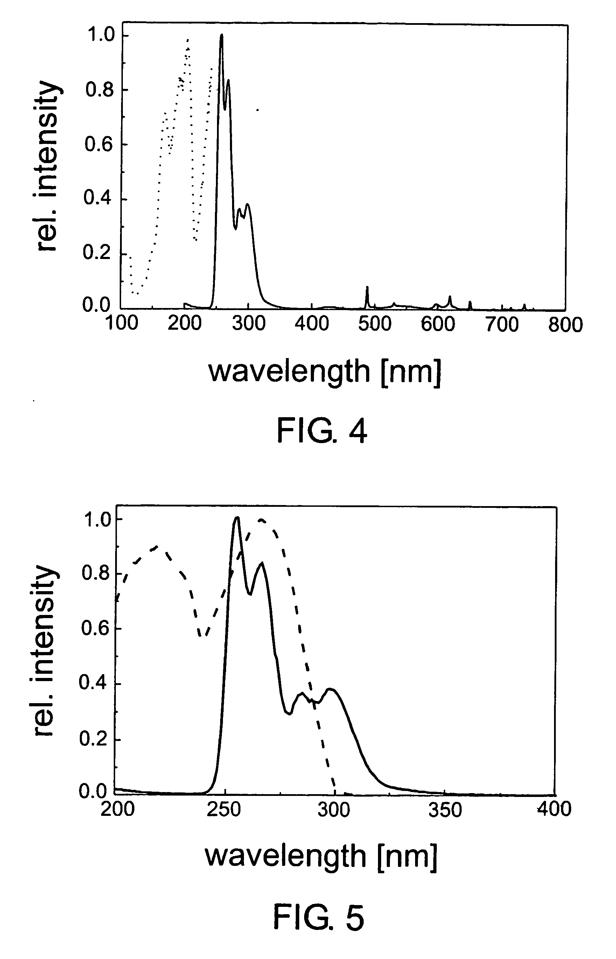

The device described, with $Ca_{1-2y}Li_2SiO_4:Pr_yNa_y$ with $0.001 \leq y \leq 0.2$ as the light-emitting compound, emits light in the UV light range, and is very suitable for high yield photolytic reactors. Fig. 4 shows the excitation and emission spectrum of the light-emitting compound. Since the emitted radiation has a narrow-band spectrum, the device in accordance with the invention may be advantageously used to undertake wavelength-selective photoreactions.

In addition, technical photochemical processes (e.g. photochlorination, photobromination, photosulfochlorination) may be undertaken more efficiently with the device in accordance with the invention.

A preferred use of this device in accordance with the invention relates to water and wastewater treatment, in which polluted water is the fluid to be treated. Examples of treatment that can be mentioned are: a) disinfection, b) degradation of pollutants, pigments or odors.

The device in accordance with the invention, with $Ca_{1-2y}Li_2SiO_4:Pr_yNa_y$ with $0.001 \leq y \leq 0.2$ as the light-emitting compound, may also be used for the sterilization of other fluids and solvents.

Phosphor $Ca_{1-2y}Li_2SiO_4:Ce_yNa_y$ with $0.001 \leq y \leq 0.2$ may also be advantageously used in a device for X-ray computer tomography.

A device for X-ray computer tomography is equipped with at least one X-ray tube and at least one X-ray detector. Usually, the device for X-ray computer tomography comprises an X-ray detector setup. An X-ray detector comprises a scintillator and a photodiode. The scintillator and photodiode are optically connected. X-rays falling onto the scintillator in the form of radiation are converted into light. The photodiode is then irradiated by the light emitted by the scintillator. As a result, information concerning the intensity of X-ray radiation can be obtained as electrical information from a photodiode.

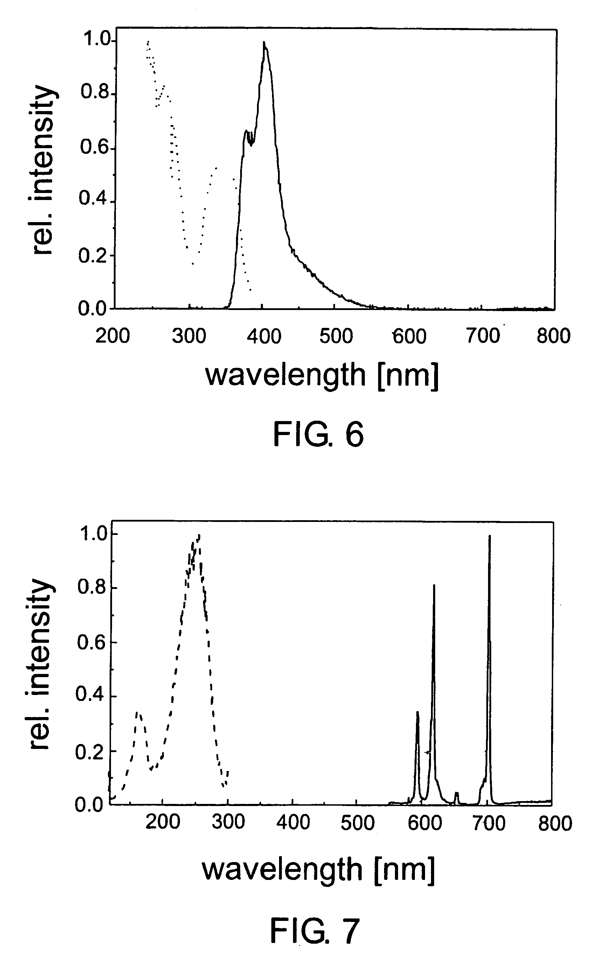

In a device in accordance with the invention for X-ray computer tomography, the scintillator comprises $Ca_{1-2y}Li_2SiO_4:Ce_yNa_y$ with $0.001 \leq y \leq 0.2$. The scintillator is generally produced by the compression method. A scintillator of this kind is secured on the photodiode using, for example, a transparent adhesive. On excitation with X-ray radiation, $Ca_{1-2y}Li_2SiO_4:Ce_yNa_y$ with $0.001 \leq y \leq 0.2$ exhibits an emission spectrum with a maximum at 400 nm. Fig. 6 shows the excitation and emission spectrum of the light-emitting compound. The phosphor is especially suitable for proving or for converting X-ray radiation since, here too, the maxima of the photomultipliers used in devices for X-ray computer tomography are based on Cs3Sb, bi-alkali (S22) or multi-alkali cathodes. Furthermore, with a decay time $\tau_{1/e}$ of 36 ns, the phosphor has a shorter decay time ($\tau_{1/e}$ = 40 ns) than the frequently used scintillator $Lu_2SiO_5$:Ce.

The light-emitting compound $Ca_{1-2y}Li_2SiO_4$:$Eu_y$$Na_y$ with $0.001 \leq y \leq 0.2$ may be advantageously used as, for example, red-emitting phosphor in a plasma screen. Fig. 7 shows the excitation and emission spectrum of the light-emitting compound.

Embodiment example 1

In order to produce $Ca_{0.98}Li_2SiO_4$:$Pr_{0.01}$,$Na_{0.01}$, we mixed 25.0 g (278 mmol) $Li_2SiO_3$, 147 mg (1.39 mmol) $Na_2CO_3$, 27.3 g (272 mmol) $CaCO_3$ and 1.21 g (2.78 mmol) $Pr(NO_3)_3$ - $6H_2O$ in demineralized $H_2O$ and suspended it. The water was removed by distillation and the residue obtained was dried. The reaction product was then annealed once in air at 700°C for 2 h, and twice in a CO atmosphere at 850°C for 12 h each time. The light-emitting compound obtained was washed multiple times with water and ethanol, dried and milled on a roller mill for several hours. The powder obtained had an average particle size of 3 $\mu$m. The absorption and emission spectrum of this light-emitting compound is shown in Fig. 4. Fig. 5 shows the DIN 5031-10 (broken line) in comparison with the emission spectrum of $Ca_{0.98}Li_2SiO_4$:$Pr_{0.01}$,$Na_{0.01}$ (solid line).

Embodiment example 2

In order to produce $Ca_{0.96}Li_2SiO_4$:$Ce_{0.02}$,$Na_{0.02}$, we mixed 9.00 g (100 mmol) $Li_2SiO_3$, 106 mg (1.00 mmol) $Na_2CO_3$, 9.61 g (96.0 mmol) $CaCO_3$ and 869 mg (2.00 mmol) $Pr(NO_3)_3 \cdot 6H_2O$ in demineralized $H_2O$ and suspended it. The water was removed by distillation and the residue obtained was dried. The reaction product was then annealed once in air at 700°C for 2 h, and twice in a CO atmosphere at 850°C for 12 h each time. The light-emitting compound obtained was washed multiple times with water and ethanol, dried and milled on a roller mill for several hours. The light-emitting compound obtained had a color point x, y of x = 0.160 and y = 0.069. The lumen equivalent of the light-emitting compound was 30 lm/W. The absorption coefficient at 254 nm of the light-emitting compound was 93% and the quantum efficiency at this wavelength was 80%. The absorption and emission spectrum for this light-emitting compound is shown in Fig. 6.

Embodiment example 3

In order to produce $Ca_{0.98}Li_2SiO_4$:$Eu_{0.01}$,$Na_{0.01}$, we mixed 20.1 g (218 mmol) $Li_2SiO_3$, 90.0 mg (2.18 mmol) NaF, 12.0 g (214 mmol) CaO and 933 mg (2.18 mmol) $Eu(NO_3)_3 \cdot 5H_2O$ in demineralized $H_2O$ and suspended it. The water was removed by distillation and the residue obtained was dried. The reaction product was then annealed once in air at 700°C for 2 h, and once at 850°C for 12 h. The light-emitting compound obtained was ground in a mortar and again annealed at 850°C for 6h. The absorption and emission spectrum for this light-emitting compound is shown in Fig. 7.

Embodiment example 4

In order to produce $Ca_{0.98}Li_2SiO_4$:$Tb_{0.01}$,$Na_{0.01}$, we mixed 20.1 g (218 mmol) $Li_2SiO_3$, 90.0 mg (2.18 mmol) NaF, 12.0 g (214 mmol) CaO and 987 mg (2.18 mmol) $Tb(NO_3)_3 \cdot 6H_2O$ in demineralized $H_2O$ and suspended it. The water was removed by distillation and the residue obtained was dried. The reaction product was then annealed once in air at 700°C for 2 h, and once at 850°C for 12 h. The light-emitting compound obtained was ground in a mortar and again annealed at 850°C for 6h.

Embodiment example 5

In order to produce $Ca_{0.98}Li_2SiO_4$:$Nd_{0.01}$,$Na_{0.01}$, we mixed 9.82 g (109 mmol) $Li_2SiO_3$, 46.0 mg (1.09 mmol) NaF, 6.00 g (107 mmol) CaO and 480 mg (1.09 mmol) $Nd(NO_3)_3 \cdot 6H_2O$ in demineralized $H_2O$ and suspended it. The water was removed by distillation and the residue obtained was dried. The reaction product was then annealed once in air at 700°C for 2 h, and once at 850°C for 12 h. The light-emitting compound obtained was ground in a mortar and again annealed at 850°C for 6h.

Embodiment example 6

Firstly, a suspension of $Ca_{0.98}Li_2SiO_4$:$Pr_{0.01}$,$Na_{0.01}$ was produced in butyl acetate with nitrocellulose as the binder. The suspension was applied to the internal walls of a tube made of synthetic quartz (Suprasil™) with an inside diameter of 5 mm using a flow coating method. The depth of coating 3 corresponded to a mass-per-unit-area for the light-emitting compound of 3 mg/cm$^2$. The binder was burnt out at a temperature below 580°C. The device was filled with xenon at a gas pressure of 200 to 300 mbar and then sealed. Two electrodes 4, 5 made of aluminum foil, were bonded diagonally onto the outside walls of the device.

The device was operated with alternating current with a square-wave characteristic at an amplitude of 6 kV and a frequency of 25 kHz.

Embodiment example 7

The discharge vessel 1 of the device in accordance with embodiment example 2 comprised a cylindrical tube made of synthetic quartz (Suprasil™) with a thickness of 0.7 mm and a diameter of 50 mm. It was filled with xenon at a pressure of 200 mbar. A spirally wound inner electrode 4 made of metal wire was arranged in the axis of the tube. Six strips of silver film were bonded onto the outside walls of the discharge vessel, in parallel with the inner electrode, as the outer electrodes 5. The inside surface of the outside walls was coated, wherein coating 3 comprised $Ca_{0.98}Li_2SiO_4$: $Pr_{0.01},Na_{0.01}$ as the light-emitting compound.

The device was operated with alternating current with a square-wave characteristic at an amplitude of 6 kV and a frequency of 25 kHz.

Embodiment example 8

The discharge vessel 1 of the device in accordance with embodiment example 8 comprised two coaxial tubes made of synthetic quartz (Suprasil™) with a thickness of 0.7 mm, an inside diameter of 50 mm and an outside diameter of 40 mm respectively. The two tubes were joined together at both ends in such a way that they formed a gas-tight hollow sleeve. Discharge vessel 1 was filled with xenon at a pressure of 200 mbar. The wall of the hollow sleeve facing the tube axis was covered, on its side facing the tube axis, with a layer of aluminum, forming the first electrode 4, and onto the wall of the hollow sleeve facing away from the tube axis, on its side facing away from the tube axis, were bonded six strips of silver film, parallel with the tube axis, forming the second electrode 5. The internal surfaces of the hollow sleeve were coated, wherein coating 3 comprised $Ca_{0.98}Li_2SiO_4$:$Pr_{0.01},Na_{0.01}$ as the light-emitting compound.

The device was operated with alternating current with a square-wave characteristic at an amplitude of 6 kV and a frequency of 25 kHz.

**Claims**

1. A device for generating radiation by means of excimer discharge, equipped with an at least partly UV-transparent discharge vessel (1), the discharge space (2) of which is filled with a gas filling, with means for igniting and maintaining an excimer discharge (4, 5) in the discharge space, and with a coating (3) comprising a light-emitting compound of the following composition:

$$(Ca_{1-x-2y}Sr_x)Li_2Si_{1-z}Ge_zO_4:Ln_yM_y,$$

   wherein Ln is a cation selected from the group $Ce^{3+}$, $Pr^{3+}$, $Sm^{3+}$, $Eu^{3+}$, $Gd^{3+}$, $Tb^{3+}$, $Dy^{3+}$, $Er^{3+}$, $Tm^{3+}$ and $Yb^{3+}$, and M is a cation selected from the group $Na^+$, $K^+$ and $Rb^+$, with
   $0 \leq x \leq 0.1$, $0.001 \leq y \leq 0.2$ and $0 \leq z \leq 1$.

2. A device as claimed in claim 1, **characterized in that** the coating (3) is equipped with a light-emitting compound of the following composition:
   $Ca_{1-2y}Li_2SiO_4:Pr_yNa_y$ with $0.001 \leq y \leq 0.2$.

3. A use of a device as claimed in claim 1 or 2 for disinfection purposes.

4. A use of a device as claimed in claim 1 or 2 for disinfecting water, air, or surfaces.

5. A light-emitting compound of the following composition:
   $(Ca_{1-x-2y}Sr_x)Li_2Si_{1-z}Ge_zO_4:Ln_yM_y,$
   wherein Ln is a cation selected from the group $Ce^{3+}$, $Pr^{3+}$, $Sm^{3+}$, $Eu^{3+}$, $Gd^{3+}$, $Tb^{3+}$, $Dy^{3+}$, $Er^{3+}$, $Tm^{3+}$ and $Yb^{3+}$, and M is a cation selected from the group $Na^+$, $K^+$ and $Rb^+$, with
   $0 \leq x \leq 0.1$, $0.001 \leq y \leq 0.2$ and $0 \leq z \leq 1$.

**6.** A light-emitting compound of the following composition:
$Ca_{1-2y}Li_2SiO_4:Pr_yNa_y$ with $0.001 \leq y \leq 0.2$.

**Patentansprüche**

**1.** Vorrichtung zur Erzeugung von Strahlung mittels einer Excimer-Entladung, ausgerüstet mit einem zumindest teilweise UV-transparenten Entladungsgefäß (1), dessen Entladungsraum (2) mit einer Gasfüllung gefüllt ist, mit Mitteln zur Zündung und Aufrechterhaltung einer Excimer-Entladung (4,5) in dem Entladungsraum und mit einer Beschichtung (3), die eine Licht emittierende Verbindung der folgenden Zusammensetzung , enthält:
$(Ca_{1-x-2y}Sr_x)Li_2Si_{1-z}Ge_zO_4:Ln_yM_y$,
wobei Ln ein aus der Gruppe $Ce^{3+}$, $Pr^{3+}$, $Sm^{3+}$, $Eu^{3+}$, $Gd^{3+}$, $Tb^{3+}$, $Dy^{3+}$, $Er^{3+}$, $Tm^{3+}$ und $Yb^{3+}$ ausgewähltes Kation und M ein aus der Gruppe $Na^+$, $K^+$ und $Rb^+$ ausgewähltes Kation ist, mit
$0 \leq x \leq 1, 0,001 \leq y \leq 0,2$ und $0 \leq z \leq 1$.

**2.** Vorrichtung gemäß Anspruch 1, **dadurch gekennzeichnet, dass** die Beschichtung (3) eine Licht emittierende Verbindung der folgenden Zusammensetzung aufweist:
$Ca_{1-2y}Li_2SiO_4:Pr_yNa_y$ mit $0,001 \leq y \leq 0,2$.

**3.** Verwendung einer Vorrichtung gemäß Anspruch 1 oder 2 zu Desinfektionszwecken.

**4.** Verwendung einer Vorrichtung gemäß Anspruch 1 oder 2 zur Desinfektion von Wasser, Luft oder Oberflächen.

**5.** Licht emittierende Verbindung der folgenden Zusammensetzung
$(Ca_{1-x-2y}Sr_x)Li_2Si_{1-z}Ge_zO_4:Ln_yM_y$,
wobei Ln ein aus der Gruppe $Ce^{3+}$, $Pr^{3+}$, $Sm^{3+}$, $Eu^{3+}$, $Gd^{3+}$, $Tb^{3+}$, $Dy^{3+}$, $Er^{3+}$, $Tm^{3+}$ und $Yb^{3+}$ ausgewähltes Kation und M ein aus der Gruppe $Na^+$, $K^+$ und $Rb^+$ ausgewähltes Kation ist, mit
$0 \leq x \leq 0,1$, $0,001 \leq y \leq 0,2$ und $0 \leq z \leq 1$.

**6.** Licht emittierende Verbindung der folgenden Zusammensetzung:
$Ca_{1-2y}Li_2SiO_4:Pr_yNa_y$ mit $0,001 \leq y \leq 0,2$.

**Revendications**

**1.** Dispositif pour générer du rayonnement au moyen d'une décharge d'excimères qui est équipé d'un récipient à décharge (1) étant au moins partiellement transparent aux rayons ultraviolets dont l'espace de décharge (2) est rempli d'un remplissage de gaz, de moyens pour amorcer et pour maintenir une décharge d'excimères (4, 5) dans l'espace de décharge et d'une couche de revêtement (3) comprenant un composé de sortie de lumière ayant la composition suivante:
$(Ca_{1-x-2y}Sr_x)Li_2Si_{1-z}Ge_zO_4:Ln_yM_y$,
où Ln est un cation qui est sélectionné parmi le groupe $Ce^{3+}$, $Pr^{3+}$, $Sm^{3+}$, $Eu^{3+}$, $Gd^{3+}$, $Tb^{3+}$, $Dy^{3+}$, $Er^{3+}$, $Tm^{3+}$ et $Yb^{3+}$ et M est un cation qui est sélectionné parmi le groupe $Na^+$, $K^+$ et $Rb^+$,
$0 \leq x \leq 0,1$, $0,001 \leq y \leq 0,2$ et $0 \leq z \leq 1$.

**2.** Dispositif selon la revendication 1, **caractérisé en ce que** la couche de revêtement (3) est équipée d'un composé de sortie de lumière ayant la composition suivante:
$Ca_{1-2y}Li_2SiO_4:Pr_yNa_y$ avec $0,001 \leq y \leq 0,2$.

**3.** Utilisation d'un dispositif selon la revendication 1 ou 2 à des fins de désinfection.

**4.** Utilisation d'un dispositif selon la revendication 1 ou 2 pour désinfecter de l'eau, de l'air ou des surfaces.

**5.** Composé de sortie de lumière ayant la composition suivante:
$Ca_{1-x-2y}Sr_x)Li_2Si_{1-z}Ge_zO_4:Ln_yM_y$
où Ln est un cation qui est sélectionné parmi le groupe $Ce^{3+}$, $Pr^{3+}$, $Sm^{3+}$, $Eu^{3+}$, $Gd^{3+}$, $Tb^{3+}$, $Dy^{3+}$, $Er^{3+}$, $Tm^{3+}$ et $Yb^{3+}$ et M est un cation qui est sélectionné parmi le groupe $Na^+$, $K^+$ et $Rb^+$ avec
$0 \leq x \leq 0,01$, $0,001 \leq y \leq 0,2$ et $0 \leq z \leq 1$.

6. Composé de sortie de lumière ayant la composition suivante:
   $Ca_{1-2y}Li_2SiO_4:Pr_yNa_y$ avec $0,001 \leq y \leq 0,2$.

FIG. 1

FIG. 2

FIG. 3

FIG. 4

FIG. 5

FIG. 6

FIG. 7